# EUROPEAN PATENT APPLICATION

(11) **EP 0 629 698 A1**
(43) Date of publication of application: **21.12.1994**
(21) Application number: 93307844.6
(22) Date of filing: 01.10.1993
(51) Int. Cl.: C12N 15/76, C12N 1/21, C12R 1/465

(54) **DNA sequences conferring hypertransposability and vectors contructed therewith**

(30) Priority: 08.10.1992 US 959938
(71) Applicant: ELI LILLY AND COMPANY, Indianapolis, Indiana 46285 (US)
(72) Inventor: Baltz, Richard Henry, Indianapolis, Indiana 46220 (US); Solenberg, Patricia Jean, Indianapolis, Indiana 46256 (US)
(74) Representative: Hudson, Christopher Mark

(57) **Abstract**

IR-L5099-10 confers hypertransposing activity on transposable elements comprising IR-L5099-10. IR-L5099-10 is a57 base pair sequence generated by a spontaneous mutation in TN5099-7. Vectors comprising IR-L5099-10 are useful in a variety of bacteria including but not limited to *Streptomyces, Chainia, Saccharopolyspora, Steptoverticillium* and *Mycobacterium.*

## Description

Transposable elements, such as transposons, insertion sequences, and transposing bacteriophages, are found in both prokaryotic and eukaryotic cells. These elements are capable of insertion events at few or many different sites along the genome. This ability to transpose and insert, even in the absence of DNA homology, has allowed transposable elements to be exploited as powerful genetic tools. Some examples of the utility of such elements include gene mapping, use as promoter probes, construction of insertion mutations, the mediation of intergeneric recombination, the insertion of genetic information into the genome of a recipient strain, and the genetic tagging of specific genes.

The development and exploitation of recombinant DNA technology in *Streptomyces* and related genera depends upon the availability of genetic tools for the introduction of foreign DNA or of multiple copies of native DNA into the genome of these cells. This development has been retarded by the very low number of transposable elements presently available for use in *Streptomyces* and related genera such as *Streptoverticillium, Saccharopolyspora, Chainia* and *Mycobacteria*.

The transposable elements available for utilization in *Streptomyces* include: IS110 (Bruton, C. and K. Chater, 1987, Nucleic Acids Res., **15**:7053-7065); 1S281 (Sladkova, A. 1986 Mol. Biol., **20**:878-881); Tn4556 (Chung, S. 1987 J. Bacteriol., **169**:4436-4441); the 2.6 kilobase minicircle from *Streptomyces coelicolor* (Lydiate, D., *et al.,* 1986, Mol. Gen. Genet., **203**:79-88) and IS493 and transposons derived therefrom.

Insertion sequence IS493 was isolated from *Streptomyces lividans* using a transposon trap. Solenberg, P. and S. Burgett, 1989, J. Bacteriol., **171**: 4807-4813. A variety of transposons have been developed utilizing IS493. *See* Solenberg, P. and S. Burgett,1989, J. Bacteriol., **171**:4807-4813; McHenney, M. and R. Baltz, 1991, J. Bacteriol., **173**: 5578-5581; Hahn, D. *et al*, 1991, J. Bacteriol., **173**:5573-5577; and Solenberg, P. and R. Baltz 1991 J. Bacteriol., **173**:1096-1104. The aforementioned transposons have proven to be useful in genetic manipulations of the various *Actinomycetales.* Transposons derived from IS493 exhibit transposition frequencies of about 10⁻⁶.

Transposon Tn5099, a xylE promoter probe transposon, which was described in Hahn *et al., supra,* was modified to comprise a promoter driving constitutive production of xylE. Transposon Tn5099-7 is the designation for the Tn5099 derivative containing the constitutively expressed xylE gene. The constitutive expression of xylE resulted in a selective pressure against xylE production and ultimately led to a deletion in the region of the xylE promoter and coding sequence. The deletion included a portion of the 5' inverted repeat of Tn5099-7 and generated a modified inverted repeat with changes in the 50-57 base area (reading left to right from the sense strand). The altered 5' inverted repeat is designated IR-L5099-10 and confers hypertransposability on transposons and other elements comprising IR-L5099-10.

IR-L5099-10, an inverted repeat generated by a spontaneous deletion in Tn5099-7, confers hypertransposing activity on transposable elements constructed therewith.

### Brief Description of the Figures

For the purposes of this disclosure, the Figures are not drawn exactly to scale.

Figure 1 -- the restriction site and function map of plasmid pCZA141.

Figure 2 -- the restriction site and function maps of Tn5096 and Tn5097.

Figure 3-- the restriction site and function map of Tn5099-10.

For purposes of the present invention, as disclosed and claimed herein, the following terms are defined below.

Am^{R} - the apramycin-resistant phenotype or gene conferring same. The Am^{R} gene is also designated *aac(3)IV*.

Ap^{R} - the ampicillin-resistant phenotype or gene conferring same.

Gene - a DNA segment which encodes a promoter function, a ribosome binding site and a translatable gene product.

Hm^{R} - the hygromycin-resistant phenotype or gene conferring same.

Hypertransposition - transposition by a transposable element at a frequency of 10⁻⁵ or greater.

Insertion sequence - small (generally less than 2000 bp) elements which encode no known genes unrelated to insertion function.

Inverted Repeats - DNA sequences at the termini of insertion sequences and transposons which facilitate insertion of the insertion sequence or transposon into host cell DNA. Inverted repeats are abbreviated IR and are sometimes designated IR-L and IR-R to denote which termini of the insertion sequence or transposon they define with IRL being the left (5') repeat and IR-R being the right (3') repeat.

Ori - an origin of replication.

Origin of replication - a DNA sequence which allows the replication of an extrachromosomal element in a specific host cell.

Recombinant DNA cloning vector - any autonomously replicating agent, including, but not limited to, plasmids and phages, comprising a DNA molecule to which one or more additional DNA segments can be or have been added.

Selectable marker - a gene or combination of genes of known function, the expression of which can be easily determined.

Transformation - the introduction of DNA into a recipient host cell.

Transposable element - a transposon, an insertion sequence, or a discrete piece of double stranded DNA which is capable of insertion at few or many sites in a genome.

Transposon - a transposable element, generally larger than 2 kb that may contain additional determinants unrelated to insertion function such as antibiotic or heavy metal resistance-conferring genes or biosynthetic genes.

The present invention provides a novel sequence conferring hypertransposing properties and is designated IR-L5099-10. Tn5099-10 comprises IR-L5099-10. Tn5099-10 is illustrative of the transposons which can be constructed using the IR-L5099-10 hypertransposing sequence of the present invention. Tn5099-10 and other vectors comprising the hypertransposing sequence of the present invention are useful for insertionally inactivating chromosomal genes as well as for inserting any sort of foreign DNA into the Streatomyces genome, gene-mapping, promoter probe construction, construction of insertion mutations, and the like. Another aspect of this invention is the use of this transposable element to create other novel transposons. The invention therefore helps to fulfill the long-felt need for such discrete, clearly defined elements for use in *Streptomyces,* thereby releasing the skilled artisan from reliance upon extra-chromosomal elements or homologous integration. Surprisingly, the IR-L5099-10 element results in transposition frequencies approximately 10³ fold higher than other transposons derived from IS493, including Tn5096 and Tn5099, and thus provides a means for facile achievement of higher transposition frequencies of genes of interest into host organisms.

The steady state transposition frequency of Tn5099- 10 was determined as described in Youngman, Philip, Plasmids:A Practical Approach (1987) K. Hardy, Ed., IRL Press, Inc., Oxford, U.K. at pages 79-103 with modifications for growth in *Streptomyces*. Three replicate experiments using a temperature sensitive delivery vector were performed. The hypertransposing activity of Tn5099-10 was demonstrated by comparison to Tn5096 and Tn5099 under equivalent experimental conditions. The results are summarized in Table 1.

**Table 1**

| Transposon | Transposition Frequency |
|---|---|
| Tn5096 | 4.3 x 10⁻⁶ |
| Tn5099 | less than 10⁻⁶ |
| Tn5099-10 | 2.5 x 10⁻³ |

The experiments summarized above demonstrate that IR-L5099-10 confers a transposition frequency approximately one thousand times greater than conventional IS493 based vectors. Skilled artisans will appreciate that the thousand fold higher transposition frequency affords a facile means for increasing gene dosage (the number of copies of a given gene) in a cell and as such would be of immense benefit in improving production yields of metabolites such as antibiotics or immunosuppressives by increasing the gene dosage of the rate limiting enzyme in the biosynthetic pathway of the metabolite.

Tn5099-10 is similar to Tn5099, which has been described in the scientific literature. *See* Hahn, R. *et al.*, 1991, J. Bact., **173**:5573-5577. Tn5099 was constructed by adding a xylE coding sequence, a hygromycin resistance gene and multiple cloning sites to IS493. Tn5099 also contains a phage lambda pL promoter positioned 5' to the hygromycin resistance gene sequence to effect transcription of the hygromycin resistance gene product in *E. coli,* thereby enabling facile identification of *E. coli* transformants. The nucleotide sequence of IS493 is taught in Solenberg, P. and Burgett, S. J., 1991, Bact. **171**:4807-4813.

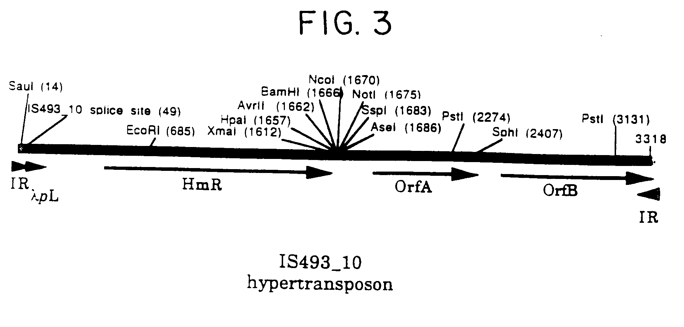

A restriction site and function map of Tn5099-10 is provided in Figure 3. The inverted repeats located at the termini of Tn5099-10 are in opposite orientation as indicated by the arrows of Figure 3. A lambda pL promoter is positioned 5' to the hygromycin resistance gene. The hygromycin resistance gene comprises its homologous promoter, which is operable in *Actinomycetes*. The phage lambda promoter was added for selection of *E. coli* transformants. The two open reading frames (OrfA and OrfB) are described in Solenberg and Burgett, *supra,* The translation product of OrfB is thought to be a transposase. The function of the OrfA gene product remains unelucidated. The OrfA functionality is not essential for transposition of IS493 based transposons.

The nucleotide sequence of Tn5099-10 is provided below as Sequence ID No:1.

GAGCGTTTTT CAACCTCAGG CTGACGGGGT CAGGGGTCAG AGGCGAGGTA
ACCATCTGCG GTGATAAATT ATCTCTGGCG GTGTTGACAT AAATACCACT
GGCGGTGATA CTGAGCACAT CAGATCGGTC GTCGAGGTCC ACAGGCCCTC
GGCGACGTTC CGCCGGGCCT CGGCGACCGC CGCGTCGAGG CGCCGGTCGA
AGGGGCAGTC CTCCACGGGC AGCTCGTGGA GGGCGCGGGC CAGCTCCGCC
ATCGCCTCGA CCACGGCGAA CCGCTGGTGC TCGGGCCACT CCTCGGCCGC
CGCGACGCCG GGGACGGCCT CCGTGACGAG CCACGCGGCG GTGTCGTCGG
CACCGCGCTC GACGACGCGG GGGACGGGGA TCGGCGGGGC CTGGCGGCGC
CTCGCCGTCG CAGAACCAGG CGGTGGCGTA CACCGTCGCC TCGGTCGGCC
CGTAGAGATT GGCGATCCCG ACCGCAGCAC CACCGAGAAC GTCCCCGACG
TGGCCGACCA GCCCGTCATC GTCAACGCCT GACCGCGGTG CGGACAGGCC
GTGTCGCGAC CGGCCGTGCG GAATTAAGCC GGCCCGTACC CTGTGAATAG
AGGTCCGCT GTG ACA CAA GAA TCC CTG TTA CTT CTC GAC CGT
ATT GAT TCG GAT GAT TCC TAC GCG AGC CTG CGG AAC GAC CAG
GAA TTC TGG GAG CCG CTG GCC CGC CGA GCC CTG GAG GAG CTC
GGG CTG CCG GTG CCG CCG GTG CTG CGG GTG CCC GGC GAG AGC
ACC AAC CCC GTA CTG GTC GGC GAG CCC GAC CCG GTG ATC AAG
CTG TTC GGC GAG CAC TGG TGC GGT CCG GAG AGC CTC GCG TCG
GAG TCG GAG GCG TAC GCG GTC CTG GCG GAC GCC CCG GTG CCG
GTG CCC CGC CTC CTC GGC CGC GGC GAG CTG CGG CCC GGC ACC
GGA GCC TGG CCG TGG CCC TAC CTG GTG ATG AGC CGG ATG ACC
GGC ACC ACC TGG CGG TCC GCG ATG GAC GGC ACG ACC GAC CGG
AAC GCG CTG CTC GCC CTG GCC CGC GAA CTC GGC CGG GTG CTC
GGC CGG CTG CAC AGG GTG CCG CTG ACC GGG AAC ACC GTG CTC

ACC CCC CAT TCC GAG GTC TTC CCG GAA CTG CTG CGG GAA CGC
CGC GCG GCG ACC GTC GAG GAC CAC CGC GGG TGG GGC TAC CTC
TCG CCC CGG CTG CTG GAC CGC CTG GAG GAC TGG CTG CCG GAC
GTG GAC ACG CTG CTG GCC GGC CGC GAA CCC CGG TTC GGC CAC
GGC GAC CTG CAC GGG ACC AAC ATC TTC GTG GAC CTG GCC GCG
ACC GAG GTC ACC GGG ATC GTC GAC TTC ACC GAC GTC TAT GCG
GGA GAC TCC CGC TAC AGC CTG GTG CAA CTG CAT CTC AAC GCC
TTC CGG GGC GAC CGC GAG ATC CTG GCC GCG CTG CTC GAC GGG
GCG CAG TGG AAG CGG ACC GAG GAC TTC GCC CGC GAA CTG CTC
GCC TTC ACC TTC CTG CAC GAC TTC GAG GTG TTC GAG GAG ACC
CCG CTG GAT CTC TCC GGC TTC ACC GAT CCG GAG GAA CTG GCG
CAG TTC CTC TGG GGG CCG CCG GAC ACC GCC CCC GGC GCC TGA
CGC CCC GGG TGA AGA GCC CCGTCGGGCG GTGCCTGACG GGGCTTCTCA
GTTAACCTAG GATCCATGGC GGCCGCAATA TTAATCAGGC TGACGGGGTC
AGGGGTCAGA GGCGGGGTCT GGATCAGCGC GAGCGGTCGT GAGGTAGTAC
CGGTTGCGCT CGAAGCTCAC TGCCGTCTAG GACAGAAGCT GACCCTAGCT
CACGCCTACC GTCGCTGCTG GCAAGCAACA TCAGCGAAAG GACCGCG ATG
CCC CAG CAG ACC AGC CCC GAG CCG GAG GGC TAC ACC ACC GTT
GCG CCC TGG GTC GTC ACC GAC GAC ACG GGG GCT TTC CTC GAC
TTC GTC ACC CAG GCA TTC GGG GGC GAG GAA CTC GGG CGG GTG
TCG ACC GAG GAG GGC TTG ATC GGT CAC GCT GAG ATC CGA GTC
GGC GAC ACC GTC GTG TTG GCC TTC GAC CGA CAC ACG GAC TGG
CCC GCC ATG CCG AGC CTG CTG CGC GTA TTT GTC GCC GAT GCG
GAC GAG GCG TTT TCC CAG GCC GTC GCA GCT GGC GGC TAT GTC

GTC ACT TCC CTG GCC GAC GAC GCC TTC GGA CAG CGC GGA GGG
CGC ATC AAG GAC CCC TTC GGC AAT ATC TGG TGG GTG GTC AGC
TGC GTT GAG GAC GTC TCT GAG GGC GAG ATG TGG AAG CGG CTG
CAG GAC CCC GTA TAC GCC GAG GCC ATG CGC GTG GCT CAG GAG
ACA CTC GAC GCC GAG CTC AGC GGT CGA CGC CAC GGG CGC AGC
AGC GCA CCC GTC AGG ACA ACC AGC TAG ACCCACGACG
TCGGGGTTCG CATGCGCCAG ACCGGTAGCC AAGGAGCGTC TTCATTCTGC
TTCGGCAGCT TGCGGGTTGG CCTGACACAG GGGTGAAGCC CCTGGTAGAT
GGGTTTTCGA CCAAGAAATC CGTCTCTACT AGAGGCTTCAC GTG CTT GTT
TAC CCC TCC GGG CTG GAC GTG TCC AGT TCT GCC CTC CGC TTC
CTC TCC GCC CGT CTG CGG GAA CAC CGC CGT GGT CTC GGC ACC
CGG TGG CGG CGC CTG AGC GCA GGT CGG CAG GCT CTG CTC GCC
CTC GCC CAT CTC CGC AAC GGT CAT CCG TAT GTG CAG CTC GCG
GCC GGG TTC GGC GTG GGC ACC ACC ACT GCC TAC CGC TAC GTC
ACC GAG GCA GCT GAG GTT CTG GCC GCC CTT GCC CCG ACG CTC
GCC GAG GCG GTC CGG GCG GCG TCG ATG AAG GCG TTC GTC CTG
CTG GAC GGG ACG CTG CTG CCG ATC GAC CGA ATC GCC GCC GAC
CGG CCT TTC TAC TCG GGC AAG CAC AAG AAA CAC GGG ATG AAC
GTACAG GTG ATC GCA GAT CCT TCC GGC CGC CTG CTG TGG GCC
TCG CCG GCC CTG CCC GGA GCC GTC CAC GAC GTC CGT GCG GCC
CGC GAG CAC GGC ATC ATC GAC ACG CTT GCC ACA GCC GAC GTC
AAC TGC TGG GCA GAC AAG GGC TAC CAG GGC GCC GGA GGC ACG
GTC CGT GTT CCC TAC CGC GGC CGG TGG GAG ACG CTC TCT GCA
GGG CAG CAG GCC GTC AAT CGA TCC CAC GCG AAG ATC CGG GCG

CTC GTC GAG CAA GCC GTC GCG ACC CTC AAG TCC TGG CGA CTC
CTT CGC AGG CTC CGT TGC TCG ACC ACC CGC GTC ACG GTC CTC
GTC CAG GCC GTC CTC TGC CTG CAT CAG GCC AGC TCA GCT TGA
GGATGGAAAACGCTC

The nucleotide sequence of Sequence ID No:1 should be read in context with the restriction site and function map of Tn5099-10, which is provided in Figure 3.

The hypertransposing activity of Tn5099-10 has been localized to the first 57 nucleotides of the 5' insertion sequence. These first 57 nucleotides of Sequence ID No:1 are designated IR-L5099-10. The region of Sequence ID No:1 which confers hypertransposing activity (IR-L5099-10) includes the sequence identified as IS493-10 splice site in Figure 3, which was generated when the xylE gene was spontaneously deleted from Tn5099-7 to generate Tn5099-10. The sequence of IR-L5099-10 is set forth below as Sequence ID No:5.

GAGCGTTTTT CAACCTCAGG CTGACGGGGT CAGGGGTCAG AGGCGAGGTA
ACCATCT

The combined teachings of Sequence ID No:l, Sequence ID No:5 and Figure 3 enable the construction of unlimited varieties of vectors which utilize the hypertransposing function of IR-L5099-10 (Sequence ID No:5). Figure 3 provides restriction endonuclease sites which allow the facile construction of IR-L5099-10 vectors from IS493 vectors by merely substituting the IR-L5099-10 sequence for the inverted repeat at the left end (as viewed in Figure 3) of IS493. Sequence ID No:l provides the nucleotide sequence of Tn5099-10, which comprises from left to right as viewed in Figure 3: the Inverted Repeat (IR-L5099-10) which is Sequence ID No:5; phage lambda's leftward promoter; a hygromycin resistance gene; a multiple cloning site; OrfA; OrfB and the pristine IR-R of IS493. The restriction site and function map of Figure 3 provides the oligonucleotide numbers (reading from left to right) which correspond to the restriction endonuclease sites and thereby renders utilization of the components of Sequence ID No:1 to generate vectors comprising Sequence ID No:5 a routine laboratory exercise. Example 1B teaches the synthesis of a linker which is readily inserted into the unique SauI site of plasmid pCZA141 (NRRL B18417) to generate pCZA141-10. In the event skilled artisans seek additional information on IS493 insertion sequences and transposons derived therefrom reference is directed to the teachings of Hahn *et al.*, *supra*; Solenberg and Baltz, *supra;* McHenney and Baltz, *supra;* and Solenberg and Burgett, *supra,* the entire teachings of which are herein incorporated by reference.

Example 1 teaches construction of transposons derived from the IS493 sequence. Vectors which transpose at higher frequency are readily prepared merely by substituting the IR-L5099-10 sequence of Sequence ID 5 for the corresponding region in vectors comprising IS493. References to literature describing Tn5096 and Tn5099 have been provided in an earlier section. Skilled artisans will appreciate the facile nature of converting such related transposons into IR-L5099-10 vectors.

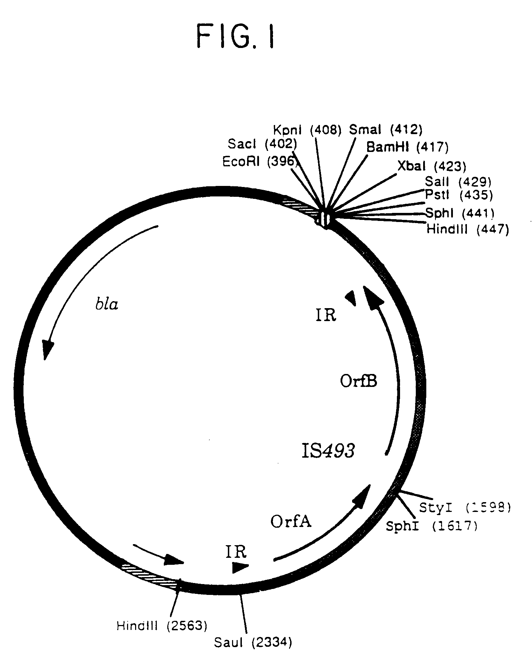

The IS493 transposable element can be obtained from plasmid pZCA141, which can be conventionally isolated from *E. coli* K12 JM109/pCZA141, a strain deposited and made part of the permanent stock culture collection of the NRRL. A culture of *E. coli* K12 JM109/pCZA141 can be obtained from the NRRL under the accession number NRRL B18417. A restriction site and function map of plasmid pCZA141 is presented in Figure 1 of the accompanying drawings.

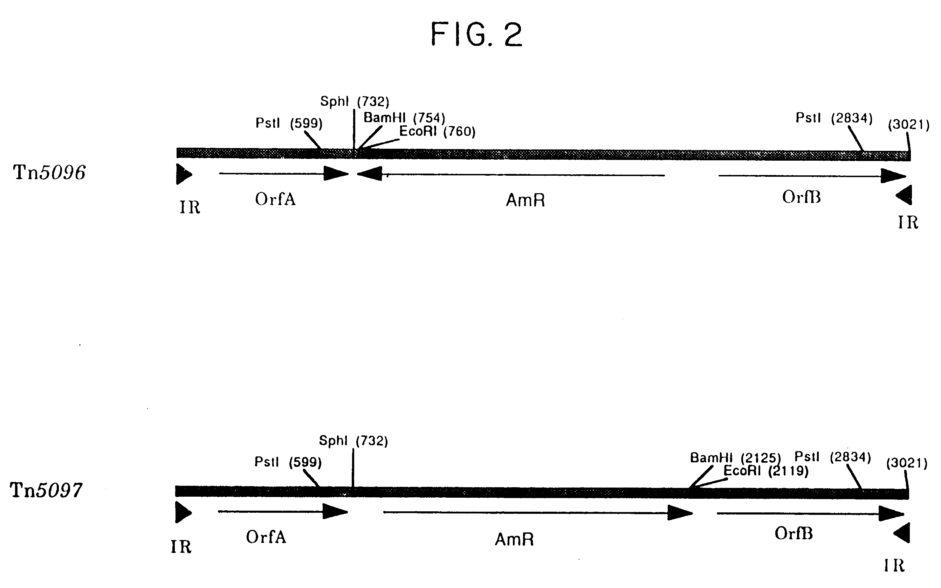

The IS493 element comprises two distinct open reading frames. The first open reading frame, designated OrfA, encodes 177 amino acids and is found between the unique SauI site and the unique SphI site. The second open reading frame, designated OrfB, encodes 233 amino acids and is located at the opposite end of the IS493 element from OrfA. The apramycin resistance-conferring gene (aac(3)IV) ligated into this site can be oriented in either direction. Tn5096, found in plasmid pCZA156, contains the aac(3)IV gene in an orientation opposite of OrfA and OrfB. Tn5097, found in plasmid pCZA157, contains the aac(3)IV gene in the same orientation as OrfA and OrfB. Both Tn5096 and Tn5097 are useful in integrating this foreign gene into *Actinomycetales* genomes. Many other novel transposons can be created by ligating any foreign gene of interest into IS493 or IR-L5099-10 based vectors. Restriction site and function maps of Tn5096 and Tn5097 are found in Figure 2 of the accompanying drawings.

Transposition events, such as those made possible by IS493 or the corresponding IR-L5099-10 vectors, are very useful because transposition may occur in a variety of locations in the genome and does not require DNA homology for integration. In addition, integration of DNA by transposition mechanisms is not limited to organisms that have functional homologous recombination systems.

The IR-L5099-10 hypertransposing sequence is in no way limited to the use in the plasmids herein described, as many different techniques and cloning vectors could be used to introduce the transposable element into the host cell. Furthermore, the types of genes integrated into the chromosome via transposition using the IR-L5099-10 element are also virtually limitless. The antibiotic resistance-conferring genes of the exemplified plasmids could be replaced by many different antibiotic resistance conferring genes or the antibiotic resistance-conferring genes could be replaced by heavy metal resistance-conferring genes, secondary metabolite biosynthetic genes, combinations thereof. The stable integration of secondary metabolite biosynthetic genes into the chromosome of a host cell can cause increased production of the desired gene product or the production of new, hybrid gene products. Those skilled in the art recognize that virtually any gene encoding any function can be transposed into the host chromosome using the IR-L5099-10 element disclosed herein. Therefore, the addition of any extraneous DNA within the parameters of the IR-L5099-10 element would be within the scope of the present invention.

Many modifications and variations of the present illustrative DNA sequence are possible. Such modifications can introduce new restriction sites into the IR-L5099-10 vectors and could greatly increase the utility of the element. Indeed, some modifications could potentially alter the frequency of transposition controlled by IS493 derivatives. These modified IR-L5099-10 elements may occur naturally such as IR-L5099-10 which generated Tn5099-10 or can be produced by synthetic methods in substantial accordance with the procedures of Itakura *et al.*, 1977, Science, 198:1056 and Crea *et al.*, 1978, Proc. Nat. Acad. Sci., USA 75:5765. In addition, synthetic components or linkers can be synthesized either by using a Systec 1450A DNA synthesizer (Systec Inc., 3816 Chandler Drive, Minneapolis, MN) or an ABS 380A DNA synthesizer (Applied Biosystems, Inc., 850 Lincoln Center Drive, Foster City, CA 94404). Many other DNA synthesizing instruments are known in the art and can be used to make synthetic DNA fragments. Therefore, the present invention is in no way limited to the DNA sequences and plasmids specifically exemplified.

Tn5099-10 resulted from the spontaneous deletion of the xylE coding region and its constitutive promoter from a transposon designated Tn5099-7. The deletion eliminated a sequence in the 50-57 base area. See Figure 3 and Sequence ID No:5. Those of ordinary skill in the art realize that further deletions in this area would lead to IRs which would likewise confer hypertransposing activity to insertion sequences and transposons. The availability of aforementioned and numerous other DNA synthesizers as well as the growing numbers of commercial laboratories which perform custom DNA synthesis and exonucleases which "chew-back" the ends of DNA sequences allows the facile preparation of IR-L5099-10-like sequences which are smaller than the IR-L5099-10 sequence of Sequence ID No:5. Accordingly, the present invention embraces functional subfragments of Sequence ID No:5.

Plasmid pCZA141-10 is illustrative of the vectors which can be constructed utilizing the IR-L5099-10 sequence of the present invention. Plasmid pCZA141-10 contains Tn5099-10 which has IR-L5099-10 as the IR-L and the IR-R of IS493. Skilled artisans realize that the IR-R of Tn5099-10 can be readily replaced with IR-L5099- 10 to generate further hypertransposing transposons. Skilled artisans also realize that the non-Tn5099-10 components of plasmid pCZA141-10 are readily interchangeable with other vector components which provide the same functions. The non-Tn5099-10 components of plasmid pCZA141-10 are derived from pUC19, a commercially available vector with numerous functional equivalents also being available from depositories such as the ATCC and the NRRL as well as numerous commercial concerns such as New England Biolabs, Pharmacia, Boehringer Mannheim, etc.. Accordingly, the vector backbone (non-Tn5099-10) is not a limitation to the vectors which can be used to introduce the IR-L5099-10 transposable elements of the present invention into host cells.

The transposable elements constructed with the IRL-5099-10 sequence are useful in numerous species and strains of *Streptomyces, Chainia, Saccharopolyspora, Steptoverticillium* and *Mycobacterium.* The transformation and transfection protocols required for introduction of vectors into the aforementioned bacteria are well known in the art and will not be reproduced as part of this disclosure.

The examples which follow are intended to further illustrate the present invention and are not to be interpreted as limiting on the scope thereof. While the examples and detailed description sections of the present invention are sufficient to guide anyone of ordinary skill in the art in the practice of the present invention, skilled artisans are also directed to Molecular Cloning A Laboratory Manual, Second Edition, Sambrook, J., Fritsch, E. F., and Maniatis, T., Cold Spring Harbor Press, 1989, and Current Protocols In Molecular Biology, Ausubel, F.M., Brent, R., Kingston, R.E., Moore, D. D., Seidman, J.G., Smith, J.A.,and Struhl, K., Ed. Greene Publishing Associates and Wiley-Interscience, 1989. The aforementioned resources provide an excellent technical supplement to any discourse in genetic engineering. *Streptomyces* and related genera such as *Saccharopolyspora, Streptoverticillium,* and *Chainia* require adaptation of the techniques described in Sambrook, *supra* and Molecular Cloning, which are directed primarily towards *E. coli* and related gram negative bacilli. Genetic Manipulation of Streptomyces-A Laboratory Manual, Hopwood, D. A., *et al.,* Ed. F. Crowe and Sons, Ltd., Norwich, U.K. provides an excellent supplement as it provides a "methods book" approach to the molecular biology protocols used in genetic manipulations of *Streptomyces.*

### Example 1

### CONSTRUCTION OF PLASMID pCZA141-10

### A. Isolation of Plasmid DCZA141

Plasmid pCZA141, which contains IS493 on an approximately 2.1 kb HindIII restriction fragment is obtained from the Northern Regional Research Center, NRRL, from *E. coli* K12 JM109/pCZA141 under the accession number NRRL B-18417. A restriction site and function map of plasmid pCZA141 is presented in Figure 1 of the accompanying drawings.

The lyophils of K12 JM109/pCZA141 are plated onto L-agar plates (10 g of Bacto-tryptone, 10 g of NaCl, 5 g of Bacto-Yeast Extract, and 15 g of agar per liter) containing 25 µg/ml ampicillin to obtain a single colony isolate of the strain. This colony is used to inoculate about 500 ml of L broth (1% (w/v) Bacto tryptone; 0.5% (w/v) yeast extract; 0.5% NaCl and 0.1% (w/v) dextrose at pH 7) containing 25 µg/ml ampicillin, and the resulting culture is incubated at 37°C with aeration until the cells reach stationary phase.

Plasmid DNA is obtained from the cells in accordance with the following procedure, which is adapted from Maniatis *et al*., 1982, Molecular Cloning (Cold Spring Harbor Laboratory). This same procedure is used, but on a smaller scale and with the ultracentrifugation steps replaced with phenol followed by chloroform extractions, to prepare the plasmid DNA used to identify the *E. coli* transformants.

About 500 ml of stationary-phase *E. coli* K12 JM109/pCZA141 cells are harvested by centrifugation at 4000Xg for 10 minutes at 4°C, and the supernatant is discarded. The cell pellet is washed in 100 ml of ice-cold STE buffer (0.1 M NaCl; 10 mM Tris-HCl, pH 7.8; and 1 mM EDTA). After the cell pellet is washed, the pellet is resuspended in 10 ml of Solution 1 (50 mM glucose; 25 mM Tris-HCl, pH=8.0; and 10 mM EDTA) that contains 5 mg/ml lysozyme and is left at room temperature for 10 minutes. Twenty ml of Solution 2 (0.2 N NaOH and 1% SDS) are then added to the lysozyme-treated cells, and the solution is gently mixed by inversion. The mixture is incubated on ice for 10 minutes.

Fifteen ml of ice-cold, 3 M sodium acetate, pH=4.8, are added to the lysed-cell mixture, and the solution is mixed by inversion. The solution is incubated on ice for 60 minutes. The 3 M sodium acetate solution is prepared by mixing equal volumes of 3 M acetic acid and 3 M sodium acetate.

The lysed cell mixture is centrifuged in a Beckman SW27 rotor (or its equivalent) at 20,000 rpm for 20 minutes at 4°C. About 36 ml of supernatant are recovered, and 2.5 volumes of ethanol are added, mixed, and the resulting solution left on ice for 15 minutes. The plasmid DNA is collected by centrifugation at 12,000Xg for 30 minutes at room temperature. The supernatant is discarded, and the DNA pellet is washed with 70% ethanol at room temperature. The ethanol wash is decanted, and the pellet is dried in a vacuum desiccator. The pellet is then resuspended in 8 ml of TE buffer.

Eight grams of CsCl are added to the DNA solution. About 0.8 ml of a 10 mg/ml solution of ethidium bromide in water are added for each 10 ml of CsCl-DNA solution. The final density of the solution is about 1.55 g/ml, and the ethidium bromide concentraton is about 800 µg/ml. The solution is transferred to a Beckman Type 50 centrifuge tube, filled to the top with TE buffer containing 1.55 g/ml CsCl, sealed, and centrifuged at 45,000 rpm for 24 hours at 20°C. After centrifugation, two bands of DNA are visible in ordinary light and become even more prominent in UV light. The cap is removed from the tube, and the lower DNA band is recovered using a syringe with a #21 hypodermic needle inserted through the side of the centrifuge tube.

The ethidium bromide is removed from the solution of plasmid DNA by several extractions with water-saturated 1-butanol, and the CsCl is removed by dialysis against TE buffer. After extractions with buffered phenol and then chloroform, the DNA is precipitated, washed with 70% ethanol, and dried. About 0.5 mg of plasmid pCZA141 DNA can be obtained by this procedure.

### B. Digestion of Plasmid pCZA141 with SauI

About 5 µg of plasmid pCZA141 are digested with SauI. The composition of digestion buffers and reaction conditions for restriction endonuclease cleavage are provided in New England Biolabs Catalogs and product literature as well as the Sambrook *et al.*, *supra*, and Molecular Cloning, *supra,* references and will not be recited here. Plasmid pCZA141 contains a single SauI site, which is near the 5' end of IS493 and within the IR-L of IS493. Digestion of plasmid pCZA141 linearizes the vector to accommodate the DNA linker which convert IS493 to Tn5099-10. Following digestion with SauI the linear pCZA141 was conventionally purified.

### C. Preparation of the Linker

Ligation of the DNA sequence provided below into the unique SauI site of plasmid pCZA141 IS493 generates Tn5099-10. 
TCAGGCTGAC GGGGTCAGGG GTCAGAGGCG AGGTAACCAT CTGCGGTGAT
AAATTATCTC TGGCGGTGTT GACATAAATA CCACTGGCGG TGATACTGAG
CACATCAGAT CGGTCGTCGA GGTCCACAGG CCCTCGGCGA CGTTCCGCCG
GGCCTCGGCG ACCGCCGCGT CGAGGCGCCG GTCGAAGGGG CAGTCCTCCA
CGGGCAGCTC GTGGAGGGCG CGGGCCAGCT CCGCCATCGC CTCGACCACG
GCGAACCGCT GGTGCTCGGG CCACTCCTCG GCCGCCGCGA CGCCGGGGAC
GGCCTCCGTG ACGAGCCACG CGGCGGTGTC GTCGGCACCG CGCTCGACGA
CGCGGGGGAC GGGGATCGGC GGGGCCTGGC GGCGCCTCGC CGTCGCAGAA
CCAGGCGGTG GCGTACACCG TCGCCTCGGT CGGCCCGTAG AGATTGGCGA
TCCCGACCGC AGCACCACCG AGAACGTCCC CGACGTGGCC GACCAGCCCG
TCATCGTCAA CGCCTGACCG CGGTGCGGAC AGGCCGTGTC GCGACCGGCC
GTGCGGAATT AAGCCGGCCC GTACCCTGTG AATAGAGGTC CGCT GTG
ACA CAA GAA TCC CTG TTA CTT CTC GAC CGT ATT GAT TCG GAT
GAT TCC TAC GCG AGC CTG CGG AAC GAC CAG GAA TTC TGG GAG
CCG CTG GCC CGC CGA GCC CTG GAG GAG CTC GGG CTG CCG GTG
CCG CCG GTG CTG CGG GTG CCC GGC GAG AGC ACC AAC CCC GTA
CTG GTC GGC GAG CCC GAC CCG GTG ATC AAG CTG TTC GGC GAG
CAC TGG TGC GGT CCG GAG AGC CTC GCG TCG GAG TCG GAG GCG
 
TAC GCG GTC CTG GCG GAC GCC CCG GTG CCG GTG CCC CGC CTC
CTC GGC CGC GGC GAG CTG CGG CCC GGC ACC GGA GCC TGG CCG
TGG CCC TAC CTG GTG ATG AGC CGG ATG ACC GGC ACC ACC TGG
CGG TCC GCG ATG GAC GGC ACG ACC GAC CGG AAC GCG CTG CTC
GCC CTG GCC CGC GAA CTC GGC CGG GTG CTC GGC CGG CTG CAC
AGG GTG CCG CTG ACC GGG AAC ACC GTG CTC ACC CCC CAT TCC
GAG GTC TTC CCG GAA CTG CTG CGG GAA CGC CGC GCG GCG ACC
GTC GAG GAC CAC CGC GGG TGG GGC TAC CTC TCG CCC CGG CTG
CTG GAC CGC CTG GAG GAC TGG CTG CCG GAC GTG GAC ACG CTG
CTG GCC GGC CGC GAA CCC CGG TTC GGC CAC GGC GAC CTG CAC
GGG ACC AAC ATC TTC GTG GAC CTG GCC GCG ACC GAG GTC ACC
GGG ATC GTC GAC TTC ACC GAC GTC TAT GCG GGA GAC TCC CGC
TAC AGC CTG GTG CAA CTG CAT CTC AAC GCC TTC CGG GGC GAC
CGC GAG ATC CTG GCC GCG CTG CTC GAC GGG GCG CAG TGG AAG
CGG ACC GAG GAC TTC GCC CGC GAA CTG CTC GCC TTC ACC TTC
CTG CAC GAC TTC GAG GTG TTC GAG GAG ACC CCG CTG GAT CTC
TCC GGC TTC ACC GAT CCG GAG GAA CTG GCG CAG TTC CTC TGG
GGG CCG CCG GAC ACC GCC CCC GGC GCC TGACGCCCCG
GGTGAAGAGC CCCGTCGGGC GGTGCCTGAC GGGGCTTCTC AGTTAACCTA
GGATCCATGG CGGCCGCAAT ATTAA (Sequence ID No:6)
Oligonucleotide synthesis is well known in the art and the sequence of IR-L5099-10 (Sequence ID No:6) is readily synthesized by such methods. See Sambrook and Maniatis, *supra,* and Current Protocols in Molecular Biology, *supra*. The linker sequence corresponds to Sequence ID No:6. The linker is synthesized as a series of oligonucleotides which collectively encompass both strands of Sequence ID No:6. The selection of appropriate oligonucleotides is a matter of choice. The present inventors did not synthesize the fragment since Tn5099-10 resulted from a spontaneous deletion in Tn5099-7 as described in an earlier section of the specification. The linker provided herein is provided to supplement the combined teachings of Sequence ID No:1 (IS493) and Sequence ID No:5 (IR-L5099-10), which are deemed enabling in and of themselves.

### D. Ligation of the 1675 bp linker into pCZA141 to generate plasmid pCZA141-10

The linearized pCZA141 of Example 1A, which has SauI overhangs and the 1675 bp linker of Example 1B, which also has SauI overhangs are ligated in accordance with procedures well known in the art. *See* Sambrook *et al., supra,* at 1.53-1.72.

### E. Transformation of E. coli MM294 with the ligation mixture of Example 1C.

To prepare *E. coli* K12 MM294 cells that are competent for transformation, the lyophils of *E. coli* K12 MM294 obtained from the NRRL (accession number NRRL B-15625) are reconstituted to isolate single colonies. One single-colony isolate of MM294 is inoculated into 5 ml of broth and the culture is incubated at 37°C overnight with aeration. Fifty µl of the overnight culture are used to inoculate 5 ml of broth. The culture is incubated at 37°C overnight with aeration. The following morning, the culture is diluted to 200 ml with broth. The diluted culture is incubated at 37°C with aeration until the absorbance at 550 nm (A) is about 0.5. The culture is cooled for ten minutes in an ice-water bath, and the cells are then collected by centrifugation at 4000Xg for 10 minutes at 4°C. The cell pellet is resuspended in 100 ml of cold 10 mM NaCl and then immediately repelleted by centrifugation. The cell pellet is resuspended in 100 ml of 30 mM CaCl and incubated on ice for 20 minutes.

The cells are again collected by centrifugation and resuspended in 10 ml of 30 mM CaCl. A one-half ml aliquot of the cells is added to the ligated DNA prepared above. The cell-DNA mixture is incubated on ice for one hour, heat-shocked at 42°C for 90 seconds, and then chilled on ice for about two minutes. The cell-DNA mixture is centrifuged, and the cell pellet is resuspended into 0.5 ml of broth in a 1.5 ml tube and incubated at 37°C for one hour.

Aliquots of the transformation mixture are plated on L-agar plates containing 100 µg apramycin/ml. The plates are incubated at 37°C overnight. Several apramycin-resistant colonies are selected and then screened by restriction enzyme analysis. A resriction site and function map of plasmid pCZA141 is provided in Figure 1. The insertion of the 1675 bp linker of Example 1B into the unique SauI site results in two different plasmids which differ only in the orientation of the linker. The plasmid having the linker in proper orientation contains Tn5099-10. Digestion of the desired plasmid with EcoRI and SauI results in DNA fragments of 3.6 kb and 2.9kb when resolved on an agarose gel. The plasmid having the linker in proper orientation and thus comprising Tn5099-10 (Sequence ID No:1) is designated pCZA141-10. Restriction endonuclease mapping is discussed in Sambrook *et al., supra,* at 1.85 *et. seq*.

## Claims

1. The hypertransposing DNA sequence of Sequence ID No:5 or a functional fragment thereof.

2. The DNA sequence of Claim 1 that is IR-L5099-10.

3. A transposable element comprising the DNA sequence of Claim 1.

4. The transposable element of Claim 3 that is a transposon.

5. The transposon of Claim 4 that is Tn5099-10.

6. A vector comprising the transposon of Claim 5.

7. A host cell containing a transposable element of Claim 3.

8. The host cell of Claim 7, said host cell being selected from the group consisting of *Streptomyces, Streptoverticullium, Saccharpolyspora and Mycobacterium.*

9. A method of introducing a gene of interest into a host cell, said method comprising transforming said host cell with a vector of Claim 6.

10. The method of Claim 9 wherein said gene of interest is a gene encoding an enzyme in the biosynthetic pathway of a secondary metabolite.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: DNA SEQUENCES CONFERRING
HYPERTRANSPOSIBILITY AND VECTORS CONSTRUCTED THEREWITH

(iii) NUMBER OF SEQUENCES: 3

(iv) CORRESPONDENCE ADDRESS:
(A) ADDRESSEE: Mr. C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO:1:

(i) SEQUENCE CHARACTERISTICS:
(A) LENGTH: 3318 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 610..1605

(ix) FEATURE:
(A) NAME/KEY: CDS
(B) LOCATION: 1854..2387

(ix) FEATURE:
(A) NAME/KEY: CDS
(B) LOCATION: 2539..3303

(xi) SEQUENCE DESCRIPTION: SEQ ID NO:1:

GAGCGTTTTT CAACCTCAGG CTGACGGGGT CAGGGGTCAG AGGCGAGGTA ACCATCTGCG 60

GTGATAAATT ATCTCTGGCG GTGTTGACAT AAATACCACT GGCGGTGATA CTGAGCACAT 120

CAGATCGGTC GTCGAGGTCC ACAGGCCCTC GGCGACGTTC CGCCGGGCCT CGGCGACCGC 180

CGCGTCGAGG CGCCGGTCGA AGGGGCAGTC CTCCACGGGC AGCTCGTGGA GGGCGCGGGC 240

CAGCTCCGCC ATCGCCTCGA CCACGGCGAA CCGCTGGTGC TCGGGCCACT CCTCGGCCGC 300

CGCGACGCCG GGGACGGCCT CCGTGACGAG CCACGCGGCG GTGTCGTCGG CACCGCGCTC 360

GACGACGCGG GGGACGGGGA TCGGCGGGGC CTGGCGGCGC CTCGCCGTCG CAGAACCAGG 420

CGGTGGCGTA CACCGTCGCC TCGGTCGGCC CGTAGAGATT GGCGATCCCG ACCGCAGCAC 480

CACCGAGAAC GTCCCCGACG TGGCCGACCA GCCCGTCATC GTCAACGCCT GACCGCGGTG 540

CGGACAGGCC GTGTCGCGAC CGGCCGTGCG GAATTAAGCC GGCCCGTACC CTGTGAATAG 600

AGGTCCGCT GTG ACA CAA GAA TCC CTG TTA CTT CTC GAC CGT ATT GAT 648
Val Thr Gln Glu Ser Leu Leu Leu Leu Asp Arg Ile Asp
1               5                   10

TCG GAT GAT TCC TAC GCG AGC CTG CGG AAC GAC CAG GAA TTC TGG GAG 696
Ser Asp Asp Ser Tyr Ala Ser Leu Arg Asn Asp Gln Glu Phe Trp Glu
    15                  20                  25

CCG CTG GCC CGC CGA GCC CTG GAG GAG CTC GGG CTG CCG GTG CCG CCG 744
Pro Leu Ala Arg Arg Ala Leu Glu Glu Leu Gly Leu Pro Val Pro Pro
30                  35                  40                  45

GTG CTG CGG GTG CCC GGC GAG AGC ACC AAC CCC GTA CTG GTC GGC GAG 792
Val Leu Arg Val Pro Gly Glu Ser Thr Asn Pro Val Leu Val Gly Glu
                50                  55                  60

CCC GAC CCG GTG ATC AAG CTG TTC GGC GAG CAC TGG TGC GGT CCG GAG 840
Pro Asp Pro Val Ile Lys Leu Phe Gly Glu His Trp Cys Gly Pro Glu
            65                  70                  75

AGC CTC GCG TCG GAG TCG GAG GCG TAC GCG GTC CTG GCG GAC GCC CCG 888
Ser Leu Ala Ser Glu Ser Glu Ala Tyr Ala Val Leu Ala Asp Ala Pro
        80                  85                  90

GTG CCG GTG CCC CGC CTC CTC GGC CGC GGC GAG CTG CGG CCC GGC ACC 936
Val Pro Val Pro Arg Leu Leu Gly Arg Gly Glu Leu Arg Pro Gly Thr
    95                  100                 105

GGA GCC TGG CCG TGG CCC TAC CTG GTG ATG AGC CGG ATG ACC GGC ACC 984
Gly Ala Trp Pro Trp Pro Tyr Leu Val Met Ser Arg Met Thr Gly Thr
110                 115                 120                 125

ACC TGG CGG TCC GCG ATG GAC GGC ACG ACC GAC CGG AAC GCG CTG CTC 1032
Thr Trp Arg Ser Ala Met Asp Gly Thr Thr Asp Arg Asn Ala Leu Leu
                130                 135                 140

GCC CTG GCC CGC GAA CTC GGC CGG GTG CTC GGC CGG CTG CAC AGG GTG 1080
Ala Leu Ala Arg Glu Leu Gly Arg Val Leu Gly Arg Leu His Arg Val
            145                 150                 155

CCG CTG ACC GGG AAC ACC GTG CTC ACC CCC CAT TCC GAG GTC TTC CCG 1128
Pro Leu Thr Gly Asn Thr Val Leu Thr Pro His Ser Glu Val Phe Pro
        160                 165                 170

GAA CTG CTG CGG GAA CGC CGC GCG GCG ACC GTC GAG GAC CAC CGC GGG 1176
Glu Leu Leu Arg Glu Arg Arg Ala Ala Thr Val Glu Asp His Arg Gly
    175                 180                 185

TGG GGC TAC CTC TCG CCC CGG CTG CTG GAC CGC CTG GAG GAC TGG CTG 1224
Trp Gly Tyr Leu Ser Pro Arg Leu Leu Asp Arg Leu Glu Asp Trp Leu
190                 195                 200                 205

CCG GAC GTG GAC ACG CTG CTG GCC GGC CGC GAA CCC CGG TTC GGC CAC 1272
Pro Asp Val Asp Thr Leu Leu Ala Gly Arg Glu Pro Arg Phe Gly His
                210                 215                 220

GGC GAC CTG CAC GGG ACC AAC ATC TTC GTG GAC CTG GCC GCG ACC GAG 1320
Gly Asp Leu His Gly Thr Asn Ile Phe Val Asp Leu Ala Ala Thr Glu
            225                 230                 235

GTC ACC GGG ATC GTC GAC TTC ACC GAC GTC TAT GCG GGA GAC TCC CGC 1368
Val Thr Gly Ile Val Asp Phe Thr Asp Val Tyr Ala Gly Asp Ser Arg
        240                 245                 250

TAC AGC CTG GTG CAA CTG CAT CTC AAC GCC TTC CGG GGC GAC CGC GAG 1416
Tyr Ser Leu Val Gln Leu His Leu Asn Ala Phe Arg Gly Asp Arg Glu
    255                 260                 265

ATC CTG GCC GCG CTG CTC GAC GGG GCG CAG TGG AAG CGG ACC GAG GAC 1464
Ile Leu Ala Ala Leu Leu Asp Gly Ala Gln Trp Lys Arg Thr Glu Asp
270                 275                 280                 285

TTC GCC CGC GAA CTG CTC GCC TTC ACC TTC CTG CAC GAC TTC GAG GTG 1512
Phe Ala Arg Glu Leu Leu Ala Phe Thr Phe Leu His Asp Phe Glu Val
                290                 295                 300

TTC GAG GAG ACC CCG CTG GAT CTC TCC GGC TTC ACC GAT CCG GAG GAA 1560
Phe Glu Glu Thr Pro Leu Asp Leu Ser Gly Phe Thr Asp Pro Glu Glu
            305                 310                 315

CTG GCG CAG TTC CTC TGG GGG CCG CCG GAC ACC GCC CCC GGC GCC 1605
Leu Ala Gln Phe Leu Trp Gly Pro Pro Asp Thr Ala Pro Gly Ala
        320                 325                 330

TGACGCCCCG GGTGAAGAGC CCCGTCGGGC GGTGCCTGAC GGGGCTTCTC AGTTAACCTA 1665

GGATCCATGG CGGCCGCAAT ATTAATCAGG CTGACGGGGT CAGGGGTCAG AGGCGGGGTC 1725

TGGATCAGCG CGAGCGGTCG TGAGGTAGTA CCGGTTGCGC TCGAAGCTCA CTGCCGTCTA 1785

GGACAGAAGC TGACCCTAGC TCACGCCTAC CGTCGCTGCT GGCAAGCAAC ATCAGCGAAA 1845

GGACCGCG ATG CCC CAG CAG ACC AGC CCC GAG CCG GAG GGC TAC ACC ACC 1895
Met Pro Gln Gln Thr Ser Pro Glu Pro Glu Gly Tyr Thr Thr
1 5 10

GTT GCG CCC TGG GTC GTC ACC GAC GAC ACG GGG GCT TTC CTC GAC TTC 1943
Val Ala Pro Trp Val Val Thr Asp Asp Thr Gly Ala Phe Leu Asp Phe
15 20 25 30

GTC ACC CAG GCA TTC GGG GGC GAG GAA CTC GGG CGG GTG TCG ACC GAG 1991
Val Thr Gln Ala Phe Gly Gly Glu Glu Leu Gly Arg Val Ser Thr Glu
35 40 45

GAG GGC TTG ATC GGT CAC GCT GAG ATC CGA GTC GGC GAC ACC GTC GTG 2039
Glu Gly Leu Ile Gly His Ala Glu Ile Arg Val Gly Asp Thr Val Val
50 55 60

TTG GCC TTC GAC CGA CAC ACG GAC TGG CCC GCC ATG CCG AGC CTG CTG 2087
Leu Ala Phe Asp Arg His Thr Asp Trp Pro Ala Met Pro Ser Leu Leu
65 70 75

CGC GTA TTT GTC GCC GAT GCG GAC GAG GCG TTT TCC CAG GCC GTC GCA 2135
Arg Val Phe Val Ala Asp Ala Asp Glu Ala Phe Ser Gln Ala Val Ala
80 85 90

GCT GGC GGC TAT GTC GTC ACT TCC CTG GCC GAC GAC GCC TTC GGA CAG 2183

Ala Gly Gly Tyr Val Val Thr Ser Leu Ala Asp Asp Ala Phe Gly Gln
95 100 105 110

CGC GGA GGG CGC ATC AAG GAC CCC TTC GGC AAT ATC TGG TGG GTG GTC 2231
Arg Gly Gly Arg Ile Lys Asp Pro Phe Gly Asn Ile Trp Trp Val Val
115 120 125

AGC TGC GTT GAG GAC GTC TCT GAG GGC GAG ATG TGG AAG CGG CTG CAG 2279
Ser Cys Val Glu Asp Val Ser Glu Gly Glu Met Trp Lys Arg Leu Gln
130 135 140

GAC CCC GTA TAC GCC GAG GCC ATG CGC GTG GCT CAG GAG ACA CTC GAC 2327
Asp Pro Val Tyr Ala Glu Ala Met Arg Val Ala Gln Glu Thr Leu Asp
145 150 155

GCC GAG CTC AGC GGT CGA CGC CAC GGG CGC AGC AGC GCA CCC GTC AGG 2375
Ala Glu Leu Ser Gly Arg Arg His Gly Arg Ser Ser Ala Pro Val Arg
160 165 170

ACA ACC AGC TAGACCCACG ACGTCGGGGT TCGCATGCGC CAGACCGGTA 2424
Thr Thr Ser
175

GCCAAGGAGC GTCTTCATTC TGCTTCGGCA GCTTGCGGGT TGGCCTGACA CAGGGGTGAA 2484

GCCCCTGGTA GATGGGTTTT CGACCAAGAA ATCCGTCTCT ACTAGAGGCT TCAC GTG 2541
Val
1

CTT GTT TAC CCC TCC GGG CTG GAC GTG TCC AGT TCT GCC CTC CGC TTC 2589
Leu Val Tyr Pro Ser Gly Leu Asp Val Ser Ser Ser Ala Leu Arg Phe
5 10 15

CTC TCC GCC CGT CTG CGG GAA CAC CGC CGT GGT CTC GGC ACC CGG TGG 2637
Leu Ser Ala Arg Leu Arg Glu His Arg Arg Gly Leu Gly Thr Arg Trp
20 25 30

CGG CGC CTG AGC GCA GGT CGG CAG GCT CTG CTC GCC CTC GCC CAT CTC 2685
Arg Arg Leu Ser Ala Gly Arg Gln Ala Leu Leu Ala Leu Ala His Leu
35 40 45

CGC AAC GGT CAT CCG TAT GTG CAG CTC GCG GCC GGG TTC GGC GTG GGC 2733
Arg Asn Gly His Pro Tyr Val Gln Leu Ala Ala Gly Phe Gly Val Gly
50 55 60 65

ACC ACC ACT GCC TAC CGC TAC GTC ACC GAG GCA GCT GAG GTT CTG GCC 2781
Thr Thr Thr Ala Tyr Arg Tyr Val Thr Glu Ala Ala Glu Val Leu Ala
70 75 80

GCC CTT GCC CCG ACG CTC GCC GAG GCG GTC CGG GCG GCG TCG ATG AAG 2829
Ala Leu Ala Pro Thr Leu Ala Glu Ala Val Arg Ala Ala Ser Met Lys
85 90 95

GCG TTC GTC CTG CTG GAC GGG ACG CTG CTG CCG ATC GAC CGA ATC GCC 2877
Ala Phe Val Leu Leu Asp Gly Thr Leu Leu Pro Ile Asp Arg Ile Ala
100 105 110

GCC GAC CGG CCT TTC TAC TCG GGC AAG CAC AAG AAA CAC GGG ATG AAC 2925
Ala Asp Arg Pro Phe Tyr Ser Gly Lys His Lys Lys His Gly Met Asn
115 120 125

GTA CAG GTG ATC GCA GAT CCT TCC GGC CGC CTG CTG TGG GCC TCG CCG 2973
Val Gln Val Ile Ala Asp Pro Ser Gly Arg Leu Leu Trp Ala Ser Pro
130 135 140 145

GCC CTG CCC GGA GCC GTC CAC GAC GTC CGT GCG GCC CGC GAG CAC GGC 3021
Ala Leu Pro Gly Ala Val His Asp Val Arg Ala Ala Arg Glu His Gly
150 155 160

ATC ATC GAC ACG CTT GCC ACA GCC GAC GTC AAC TGC TGG GCA GAC AAG 3069
Ile Ile Asp Thr Leu Ala Thr Ala Asp Val Asn Cys Trp Ala Asp Lys
165 170 175

GGC TAC CAG GGC GCC GGA GGC ACG GTC CGT GTT CCC TAC CGC GGC CGG 3117
Gly Tyr Gln Gly Ala Gly Gly Thr Val Arg Val Pro Tyr Arg Gly Arg
180 185 190

TGG GAG ACG CTC TCT GCA GGG CAG CAG GCC GTC AAT CGA TCC CAC GCG 3165
Trp Glu Thr Leu Ser Ala Gly Gln Gln Ala Val Asn Arg Ser His Ala
195 200 205

AAG ATC CGG GCG CTC GTC GAG CAA GCC GTC GCG ACC CTC AAG TCC TGG 3213
Lys Ile Arg Ala Leu Val Glu Gln Ala Val Ala Thr Leu Lys Ser Trp
210 215 220 225

CGA CTC CTT CGC AGG CTC CGT TGC TCG ACC ACC CGC GTC ACG GTC CTC 3261
Arg Leu Leu Arg Arg Leu Arg Cys Ser Thr Thr Arg Val Thr Val Leu
230 235 240

GTC CAG GCC GTC CTC TGC CTG CAT CAG GCC AGC TCA GCT TGAGGATGGA 3310
Val Gln Ala Val Leu Cys Leu His Gln Ala Ser Ser Ala
245 250 255

AAACGCTC 3318

(2) INFORMATION FOR SEQ ID NO:2:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

GAGCGTTTTT CAACCTCAGG CTGACGGGGT CAGGGGTCAG AGGCGAGGTA ACCATCT 57

(2) INFORMATION FOR SEQ ID NO:3:

(i) SEQUENCE CHARACTERISTICS:
(A) LENGTH: 1675 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: internal

(ix) FEATURE:
(A) NAME/KEY: CDS
(B) LOCATION: 595..1590

(xi) SEQUENCE DESCRIPTION: SEQ ID NO:2:

TCAGGCTGAC GGGGTCAGGG GTCAGAGGCG AGGTAACCAT CTGCGGTGAT AAATTATCTC 60

TGGCGGTGTT GACATAAATA CCACTGGCGG TGATACTGAG CACATCAGAT CGGTCGTCGA 120

GGTCCACAGG CCCTCGGCGA CGTTCCGCCG GGCCTCGGCG ACCGCCGCGT CGAGGCGCCG 180

GTCGAAGGGG CAGTCCTCCA CGGGCAGCTC GTGGAGGGCG CGGGCCAGCT CCGCCATCGC 240

CTCGACCACG GCGAACCGCT GGTGCTCGGG CCACTCCTCG GCCGCCGCGA CGCCGGGGAC 300

GGCCTCCGTG ACGAGCCACG CGGCGGTGTC GTCGGCACCG CGCTCGACGA CGCGGGGGAC 360

GGGGATCGGC GGGGCCTGGC GGCGCCTCGC CGTCGCAGAA CCAGGCGGTG GCGTACACCG 420

TCGCCTCGGT CGGCCCGTAG AGATTGGCGA TCCCGACCGC AGCACCACCG AGAACGTCCC 480

CGACGTGGCC GACCAGCCCG TCATCGTCAA CGCCTGACCG CGGTGCGGAC AGGCCGTGTC 540

GCGACCGGCC GTGCGGAATT AAGCCGGCCC GTACCCTGTG AATAGAGGTC CGCT GTG 597
Val
1

ACA CAA GAA TCC CTG TTA CTT CTC GAC CGT ATT GAT TCG GAT GAT TCC 645
Thr Gln Glu Ser Leu Leu Leu Leu Asp Arg Ile Asp Ser Asp Asp Ser
            5                   10                  15

TAC GCG AGC CTG CGG AAC GAC CAG GAA TTC TGG GAG CCG CTG GCC CGC 693
Tyr Ala Ser Leu Arg Asn Asp Gln Glu Phe Trp Glu Pro Leu Ala Arg
        20                  25                  30

CGA GCC CTG GAG GAG CTC GGG CTG CCG GTG CCG CCG GTG CTG CGG GTG 741
Arg Ala Leu Glu Glu Leu Gly Leu Pro Val Pro Pro Val Leu Arg Val
    35                  40                  45

CCC GGC GAG AGC ACC AAC CCC GTA CTG GTC GGC GAG CCC GAC CCG GTG 789
Pro Gly Glu Ser Thr Asn Pro Val Leu Val Gly Glu Pro Asp Pro Val
50                  55                  60                  65

ATC AAG CTG TTC GGC GAG CAC TGG TGC GGT CCG GAG AGC CTC GCG TCG 837
Ile Lys Leu Phe Gly Glu His Trp Cys Gly Pro Glu Ser Leu Ala Ser
                70                  75                  80

GAG TCG GAG GCG TAC GCG GTC CTG GCG GAC GCC CCG GTG CCG GTG CCC 885
Glu Ser Glu Ala Tyr Ala Val Leu Ala Asp Ala Pro Val Pro Val Pro
            85                  90                  95

CGC CTC CTC GGC CGC GGC GAG CTG CGG CCC GGC ACC GGA GCC TGG CCG 933
Arg Leu Leu Gly Arg Gly Glu Leu Arg Pro Gly Thr Gly Ala Trp Pro
        100                 105                 110

TGG CCC TAC CTG GTG ATG AGC CGG ATG ACC GGC ACC ACC TGG CGG TCC 981
Trp Pro Tyr Leu Val Met Ser Arg Met Thr Gly Thr Thr Trp Arg Ser
    115                 120                 125

GCG ATG GAC GGC ACG ACC GAC CGG AAC GCG CTG CTC GCC CTG GCC CGC 1029
Ala Met Asp Gly Thr Thr Asp Arg Asn Ala Leu Leu Ala Leu Ala Arg
130                 135                 140                 145

GAA CTC GGC CGG GTG CTC GGC CGG CTG CAC AGG GTG CCG CTG ACC GGG 1077
Glu Leu Gly Arg Val Leu Gly Arg Leu His Arg Val Pro Leu Thr Gly
                150                 155                 160

AAC ACC GTG CTC ACC CCC CAT TCC GAG GTC TTC CCG GAA CTG CTG CGG 1125
Asn Thr Val Leu Thr Pro His Ser Glu Val Phe Pro Glu Leu Leu Arg
            165                 170                 175

GAA CGC CGC GCG GCG ACC GTC GAG GAC CAC CGC GGG TGG GGC TAC CTC 1173
Glu Arg Arg Ala Ala Thr Val Glu Asp His Arg Gly Trp Gly Tyr Leu
        180                 185                 190

TCG CCC CGG CTG CTG GAC CGC CTG GAG GAC TGG CTG CCG GAC GTG GAC 1221
Ser Pro Arg Leu Leu Asp Arg Leu Glu Asp Trp Leu Pro Asp Val Asp
    195                 200                 205

ACG CTG CTG GCC GGC CGC GAA CCC CGG TTC GGC CAC GGC GAC CTG CAC 1269
Thr Leu Leu Ala Gly Arg Glu Pro Arg Phe Gly His Gly Asp Leu His
210                 215                 220                 225

GGG ACC AAC ATC TTC GTG GAC CTG GCC GCG ACC GAG GTC ACC GGG ATC 1317
Gly Thr Asn Ile Phe Val Asp Leu Ala Ala Thr Glu Val Thr Gly Ile
                230                 235                 240

GTC GAC TTC ACC GAC GTC TAT GCG GGA GAC TCC CGC TAC AGC CTG GTG 1365
Val Asp Phe Thr Asp Val Tyr Ala Gly Asp Ser Arg Tyr Ser Leu Val
            245                 250                 255

CAA CTG CAT CTC AAC GCC TTC CGG GGC GAC CGC GAG ATC CTG GCC GCG 1413
Gln Leu His Leu Asn Ala Phe Arg Gly Asp Arg Glu Ile Leu Ala Ala
        260                 265                 270

CTG CTC GAC GGG GCG CAG TGG AAG CGG ACC GAG GAC TTC GCC CGC GAA 1461
Leu Leu Asp Gly Ala Gln Trp Lys Arg Thr Glu Asp Phe Ala Arg Glu
    275                 280                 285

CTG CTC GCC TTC ACC TTC CTG CAC GAC TTC GAG GTG TTC GAG GAG ACC 1509
Leu Leu Ala Phe Thr Phe Leu His Asp Phe Glu Val Phe Glu Glu Thr
290                 295                 300                 305

CCG CTG GAT CTC TCC GGC TTC ACC GAT CCG GAG GAA CTG GCG CAG TTC 1557
Pro Leu Asp Leu Ser Gly Phe Thr Asp Pro Glu Glu Leu Ala Gln Phe
                310                 315                 320

CTC TGG GGG CCG CCG GAC ACC GCC CCC GGC GCC TGACGCCCCG GGTGAAGAGC 1610
Leu Trp Gly Pro Pro Asp Thr Ala Pro Gly Ala
            325                 330

CCCGTCGGGC GGTGCCTGAC GGGGCTTCTC AGTTAACCTA GGATCCATGG CGGCCGCAAT 1670

ATTAA 1675